# DEMANDE DE BREVET EUROPEEN

(11) **EP 1 025 817 A1**
(43) Date de publication de la demande: **09.08.2000**
(21) Numéro de dépôt: 00420028.3
(22) Date de dépôt: 08.02.2000
(51) Int. Cl.: A61F 2/36

(54) **Tige fémorale pour prothèse de hanche**

(30) Priorité: 08.02.1999 FR 9901669
(71) Demandeur: PROVIS S.A., 69008 Lyon (FR)
(72) Inventeur: Boudarel, Patrick, 69390 Charly (FR); Mesguich, Alain, 42570 Saint Heand (FR); Danan, Jean Paul, 91570 Bievres (FR); Nael, Jean Francois, 03000 Bressolles (FR); Azoulai, Jean Jacques, 42000 Saint Etienne (FR); Charlier, Pierre Henri, 42260 Saint Genest Malifaux (FR); Dumontier, Philippe, 03000 Yzeurre (FR); Djermag, Yves, 91820 Boutigny/Essone (FR)
(74) Mandataire: Dupuis, François

(57) **Abrégé**

Cette tige est remarquable en ce que le corps (1) comprend, au niveau de sa zone métaphysaire, et sur chacune de ses faces latérales (1a), au moins une rainure formée par trois segments rectilignes continus (a, b, c) convenablement orientés à savoir :
- un segment supérieur (a), de longueur réduite et orienté dans un plan sensiblement horizontal et perpendiculaire à l'axe du corps (1) de la tige pour améliorer l'ancrage en limitant les micromouvements dans le sens vertical et inférieur ;
- un segment intermédiaire (b), de plus grande longueur, et orienté d'une manière sensiblement parallèle à l'axe mécanique du fémur pour limiter les mouvements de basculement de la tige ;
- un segment inférieur (c) orienté d'une manière sensiblement parallèle à l'axe du corps (1) de la tige pour limiter les phénomènes de rotation.

## Description

L'invention se rattache au secteur technique des implants orthopédiques, notamment des prothèses de hanches.

On rappelle, d'une manière parfaitement connue, qu'une tige fémorale comprend pour l'essentiel un corps destiné à être implanté dans le canal médullaire du fémur. La partie supérieure du corps est profilée afin de correspondre sensiblement au profil anatomique du fémur, et est prolongée angulairement par un col présentant directement, ou d'une manière rapportée, une tête fémorale sous forme d'un corps sphérique, destinée à coopérer avec généralement la partie correspondante complémentaire d'un implant cotyloïdien impacté dans la cavité cotyloïdienne de l'os iliaque. Le corps de la tige est conformé pour être impacté dans le canal médullaire avec ou non un apport de ciment de scellement.

Différentes formes de réalisation ont été proposées à ce jour.

Le problème que se propose de résoudre l'invention est d'obtenir, au moment de l'impaction de la tige, un autoblocage de cette dernière par un contact étroit avec l'os spongieux dense dont le plan frontal et dans le plan sagittal afin d'obtenir une bonne stabilité primaire permettant ainsi d'impacter la tige avec ou non du ciment, en fonction de la qualité de l'articulation à restituer.

Pour résoudre un tel problème, il a été conçu et mis au point une tige fémorale pour prothèse de hanche dont le corps comprend, au niveau de sa zone métaphysaire, et sur chacune de ses faces latérales, au moins une rainure formée par trois segments rectilignes continus convenablement orientés à savoir :
- un segment supérieur, de longueur réduite et orienté dans un plan sensiblement horizontal et perpendiculaire à l'axe du corps de la tige pour améliorer l'ancrage en limitant les micro-mouvements dans le sens vertical et inférieur ;
- un segment intermédiaire de plus grande longueur et orienté d'une manière sensiblement parallèle à l'axe mécanique du fémur pour limiter les mouvements de basculement de la tige ;
- un segment inférieur orienté d'une manière sensiblement parallèle à l'axe du corps de la tige pour limiter les phénomènes de rotation.

Avantageusement, compte tenu du problème posé à résoudre chaque face latérale présente trois rainures de profondeur réduite. Les segments horizontaux des rainures sont décalés en hauteur d'une manière dégressive depuis le bord externe de la tige.

Pour résoudre le problème posé d'obtenir un contact étroit avec l'os spongieux dense sur le plan frontal et dans le plan sagittal, et de ne plus avoir de contact au niveau de la corticale diaphysaire afin d'éviter le phénomène de report de contraintes, la tige est formée, sur la totalité de sa longueur considérée à partir de très sensiblement la base du col, de trois zones tronconiques successives de section progressivement décroissante.

Pour résoudre le problème posé d'obtenir un contact optimal avec le spongieux dense, elle présente une section transversale ovoïde.

Selon une autre caractéristique de l'invention, elle présente un rayon de courbure interne combiné avec une absence de collerette d'appui pour obtenir un effet de blocage dans le spongieux et une tenue isostatique.

L'invention est exposée ci-après plus en détail à l'aide des figures des dessins annexés dans lesquels :
- la figure 1 est une vue de face de l'ensemble de la tige fémorale ;
- la figure 2 est une vue en plan correspondant à la figure 1 ;
- la figure 3 est une vue en coupe longitudinale considérée selon la ligne 3-3 ;
- la figure 4 est une vue en coupe transversale considérée selon la ligne 4-4 de la figure 1 ;
- la figure 5 est une vue en coupe transversale considérée selon la ligne 5-5 de la figure 1 ;
- la figure 6 est une vue en coupe transversale considérée selon la ligne 6-6 de la figure 1.

D'une manière parfaitement connue, la tige fémorale désignée dans son ensemble par (T), comprend un corps (1) destiné à être implanté dans le canal médullaire du fémur. Le corps (1) est prolongé angulairement au niveau de son extrémité supérieure par un col (2) disposé angulairement par rapport à l'axe général du corps (1). Le col (2), de section transversale cylindrique, est raccordé à l'extrémité supérieure du corps (1) par une embase (2a). Dans l'exemple illustré, la tige a une forme générale ergonomique ne présentant aucun angle vif, afin d'éviter les contraintes lors du transfert de charge de la tige au fémur, susceptibles de provoquer un descellement ou des douleurs. Le bord interne (1a) présente une courbure combinée avec une absence de collerette d'appui au niveau du raccordement de l'embase (2a) à l'extrémité du corps (1) pour obtenir un effet de blocage dans le spongieux et une tenue isostatique. Cette absence de collerette permet en outre de bénéficier de l'effet amortisseur du spongieux.

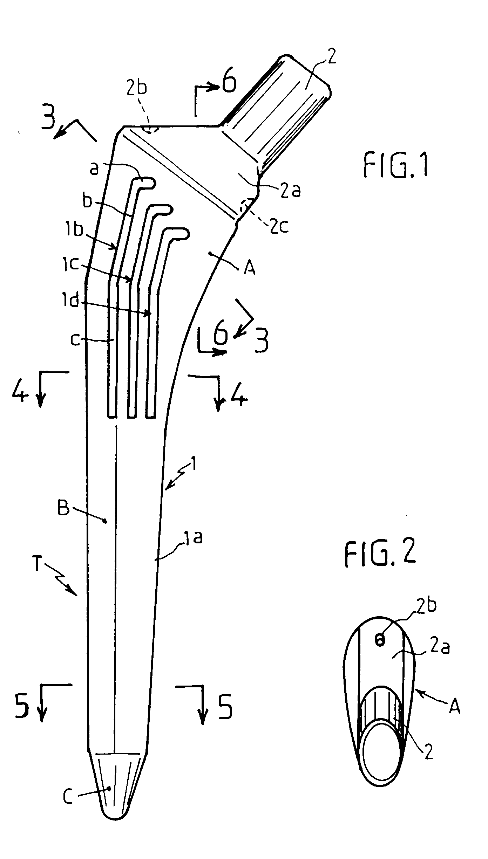

Comme le montre la figure 1, l'ensemble de la tige, notamment du corps (1), est formé sur la totalité de sa longueur considérée à partir de très sensiblement l'embase (2a) du col (2), de trois zones tronconiques successives (A, B, C), de section progressivement décroissante. Il en résulte, au niveau de l'extrémité (C) et d'une partie de la zone (B), que le corps (1) n'est plus en contact avec la corticale diaphysaire afin d'éviter le phénomène de report de contrainte.

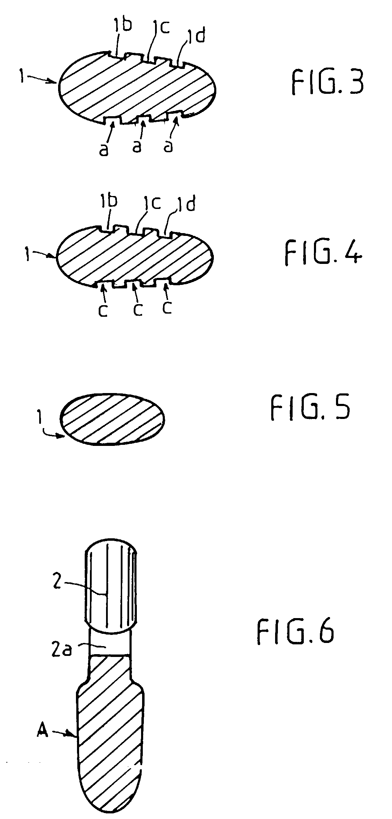

Comme le montrent les figures 3, 4 et 5, notamment, le corps (1) présente une section transversale de forme générale ovoïde afin d'obtenir un contact optimal avec le spongieux dense.

Selon une caractéristique importante de l'invention, le corps (1) comprend au niveau de sa zone métaphysaire et sur chacune de ses faces latérales, au moins une, mais de préférence trois rainures (1b, 1c, 1d) formées chacune par trois segments rectilignes convenablement orientés (a, b, c).

Le segment supérieur (a), de longueur réduite, est orienté dans un plan sensiblement horizontal et perpendiculaire à l'axe du corps (1) de la tige pour améliorer l'ancrage en limitant les micro-mouvements dans le sens vertical et inférieur. Autrement dit, ces différentes rainures horizontales (a) réduisent le phénomène à long terme d'enfoncement de la tige.

Dans une version à cimenter de la tige, ces dispositions permettent d'obtenir un ancrage de ciment chirurgical au niveau des rainures (a) limitant, comme indiqué, les micro-mouvements dans le sens vertical et inférieur, source de micro-frottements susceptibles de conduire à une altération préjudiciable du contact ciment - tige fémorale. De même, dans une version sans ciment de la tige fémorale revêtue dans ce cas d'une couche d'hydroxyapatite, on obtient un ancrage de l'os spongieux au niveau des rainures (a) en constituant comme précédemment des points d'ancrage de chaque côté, limitant les micro-mouvements dans le sens vertical et inférieur, assurant ainsi un renforcement de la stabilité primaire indispensable à une prothèse sans ciment.

Les segments intermédiaires (b) sont de plus grande longueur et orientés d'une manière sensiblement parallèle à l'axe mécanique du fémur, pour limiter les mouvements de basculement de la tige contribuant ainsi à la stabilité primaire de la prothèse. C'est le cas notamment si cette dernière est implantée sans ciment.

Dans le cas où elle est implantée avec du ciment, ses segments (b) constituent plusieurs points d'ancrage limitant avantageusement les micro-mouvements dans le sens sagittal et les micro-mouvements de rotation, source de frottements susceptibles d'entraîner, à moyen terme, un descellement de la tige.

Les segments inférieurs (c) sont orientés d'une manière sensiblement parallèle à l'axe du corps de la tige pour limiter les phénomènes de rotation. L'ensemble de ces rainures (1a, 1b, 1c), composées chacune des segments rectilignes (a, b, c) ont une profondeur réduite afin d'économiser le spongieux.

Enfin, d'une manière connue, la tige fémorale présente, notamment au niveau de son embase (2a), des orifices d'impaction (2b) et d'extraction (2c).

Les avantages ressortent bien de la description, en particulier on souligne et on rappelle :
- La qualité de l'ancrage obtenu avec ou sans ciment, compte tenu de la disposition et de l'orientation des rainures limitant les micro-mouvements du corps de la tige dans les différents plans de l'espace et en obtenant une parfaite stabilité primaire de l'ensemble de la tige.
- La forme ovoïde du corps facilite la reconstitution de l'os dans le cadre d'une implantation sans ciment et permet également la mise en place de la tige à cimenter étant donné qu'elle ne présente aucun angle droit ou arête vive susceptibles d'entraîner une usure prématurée du ciment.

## Revendications

1. Tige fémorale pour prothèse de hanche comprenant un corps (1) destiné à être implanté dans le canal médullaire du fémur et un col (2) destiné à recevoir une tête fémorale, le corps (1) comprenant, au niveau de sa zone métaphysaire, et sur chacune de ses faces latérales (1a), une série de rainures, caractérisée en ce que chaque rainure est formée par trois segments rectilignes continus (a, b, c) convenablement orientés à savoir :
- un segment supérieur (a), de longueur réduite et orienté dans un plan sensiblement horizontal et perpendiculaire à l'axe du corps (1) de la tige pour améliorer l'ancrage en limitant les micromouvements dans le sens vertical et inférieur ;
- un segment intermédiaire (b), de plus grande longueur, et orienté d'une manière sensiblement parallèle à l'axe mécanique du fémur pour limiter les mouvements de basculement de la tige ;
- un segment inférieur (c) orienté d'une manière sensiblement parallèle à l'axe du corps (1) de la tige pour limiter les phénomènes de rotation.

2. Tige fémorale selon la revendication 1, caractérisée en ce que chaque face latérale (1a) présente trois rainures de profondeur réduite (1b, 1c, 1d).

3. Tige fémorale selon la revendication 1, caractérisée en ce que les segments horizontaux (a) des rainures (1b, 1c, 1d) sont décalés en hauteur d'une manière dégressive depuis le bord externe.

4. Tige fémorale selon la revendication 1, caractérisée en ce qu'elle est formée, sur la totalité de sa longueur considérée à partir de très sensiblement la base du col (2), de trois zones tronconiques successives de section progressivement décroissante (A, B, C).

5. Tige fémorale selon la revendication 1, caractérisée en ce qu'elle présente une section transversale ovoïde.

6. Tige fémorale selon la revendication 1, caractérisée en ce qu'elle présente un rayon de courbure interne combiné avec une absence de collerette d'appui pour obtenir un effet de blocage dans le spongieux et une tenue isostatique.
